Europäisches Patentamt

European Patent Office

Office européen des brevets

(19)

(11) Publication number: **0 375 176**
**A1**

## (12) EUROPEAN PATENT APPLICATION

(21) Application number: **89312353.9**

(22) Date of filing: **28.11.89**

(51) Int. Cl.5: **A61F 2/16**

(30) Priority: **22.12.88 GB 8829970**

(43) Date of publication of application:
**27.06.90 Bulletin 90/26**

(84) Designated Contracting States:
**DE FR IT SE**

(71) Applicant: **Haworth, Stephen Michael**
**18 Arlington Drive Mapperley Park**
**Nottingham, NG3 5EN(GB)**

(72) Inventor: **Haworth, Stephen Michael**
**18 Arlington Drive Mapperley Park**
**Nottingham, NG3 5EN(GB)**

(74) Representative: **Gilding, Martin John**
**Eric Potter & Clarkson St. Mary's Court St.**
**Mary's Gate**
**Nottingham NG1 1LE(GB)**

(54) **Intraocular lens.**

(57) A lens (10) for intraocular implantation comprises an optic (12) and a haptic comprising two portions (14) positioned at opposite ends of the optic (12), the portions (14) in plan being part of an annulus centered on the centre of the optic (12) and being defined at opposite sides by parallel chords (16) of the circle defining the outer periphery (18) of the annulus. The lens (10) is relatively simple to implant while being less likely to move after implantation than some previously known lens implants.

Fig. 1

EP 0 375 176 A1

Xerox Copy Centre

## INTRAOCULAR LENS

This invention is concerned with lenses adapted to be implanted intraocularly.

When a cataract is removed, a known technique involves the replacement of a diseased lens by an artificial lens. One of the techniques used for implanting the replacement lens intraocularly is to implant the replacement lens in the remains of the capsular bag of the eye. The replacement lenses commonly comprise an optic part of the lens adapted to receive and focus light appropriately and the haptic which is arranged to support the lens. Commonly, the haptics of replacement lenses are in the form of flexible members projecting from the optic. However, because of the design of these lenses there is a tendency for them to become decentered. Furthermore, this type of implant tends to permit the remains of the anterior capsule and the posterior capsule to contact one another in places and this can result in a secondary cataract.

A further type of implant which has been proposed is in the form of a complete disc, a central region of which provides the optic: the disc type implants which have been used commonly have a smoothly curved (generally partly spherical) rear surface. However, while this type of lens has substantially prevented the anterior and posterior capsules coming into damaging contact, insertion of the disc type lens into the capsular bag is difficult because an incision in the eye sufficiently large to insert a disc of the relevant diameter must be made and the lens introduced through the pupil thus making the necessary surgery more difficult and therefore somewhat more hazardous. In order to facilitate implanting of the disc type lens, they are commonly made of smaller overall diameter than could be comfortably accommodated in the capsular bag so there is, in any event, a tendency for the disc type of lens to be displaced, to some extent, in the capsular bag or to dislocate forwardly, out of the capsular bag.

Yet another type of implant lens which has been proposed has a haptic which consists of three limbs, a first upper limb and two lower limbs symmetrically disposed relative to the upper limb but positioned such that the included angle between the centre lines of these two lower limbs is only about 67°. This results in haptics which are of considerably greater length in a vertical direction than they are wide so that they may be inserted through a much narrower incision than the disc type lens.

They are retained in position, once inserted, by virtue of their vertical length and the positioning of the two lower limbs which militates against displacement of the lens once inserted. However there is, nevertheless, still a tendency for this type of lens to sit eccentrically in the capsular bag and, furthermore, there is considerably greater opportunity for contact between the anterior and posterior capsules than is the case with the disc type of implant. A further disadvantage of the type of implant having three limbs is that the pressure distribution at opposite ends of the lens is uneven.

One of the various objects of the present invention is to provide an improved lens adapted to be implanted intraocularly.

The invention may be considered to provide, in one aspect, a lens adapted to be implanted intraocularly comprising an optic and a haptic wherein the haptic comprises two portions positioned at opposite ends of the optic said two portions viewed in plan each being part of an annulus centred on the centre of the optic and being defined at opposite sides by parallel or substantially parallel chords of the circle defining the outer periphery of the annulus.

In a preferred lens it is, of course, important that the lens be sufficiently rigid so that, when implanted, there is no significant possibility that it will flex and become dislodged from the implanted position.

Preferably the two end portions are substantially planar and are preferably generally parallel with a plane tangential to the centre of the optic.

Preferably the haptic further comprises a portion which is annular in plan and generally frusto-conical, between inner boundaries of said two end portions and the periphery of the optic. Preferably the semi-apical angle of the frusto-conical portion is between 55° and 75°.

Preferably the optic of a lens in accordance with the invention is circular in plan, suitably between 5 and 7 mm in diameter, preferably about 6 mm in diameter.

Preferably, in a lens in accordance with the invention, the diameter of the outer periphery of said two end portions is between 9.5 mm and 11.5 mm, more preferably about 10.5 mm.

In a lens in accordance with the invention the distance between said chords preferably exceeds the diameter of the optic and is suitably between about 6 mm and 9 mm, preferably between 6.5 mm and 8 mm, more preferably about 7 mm.

Preferably a lens in accordance with the invention is made of a suitable sufficiently rigid plastics material, for example polymethylmethacrylate and suitably includes an ultraviolet light filter.

Means to facilitate implanting the lens, for example holes of appropriate location and diameter, are suitably provided in said two portions.

There now follows a detailed description to be read with reference to the accompanying drawings of a lens adapted to be implanted intraocularly. It will be realised that this lens has been selected for description to illustrate the invention by way of example.

In the accompanying drawings:-

Figure 1 is a plan view showing a lens embodying the invention;

Figure 2 is a view in section on the line II-II of Figure 1; and

Figure 3 is a sectional view through an eye showing the illustrative lens in place.

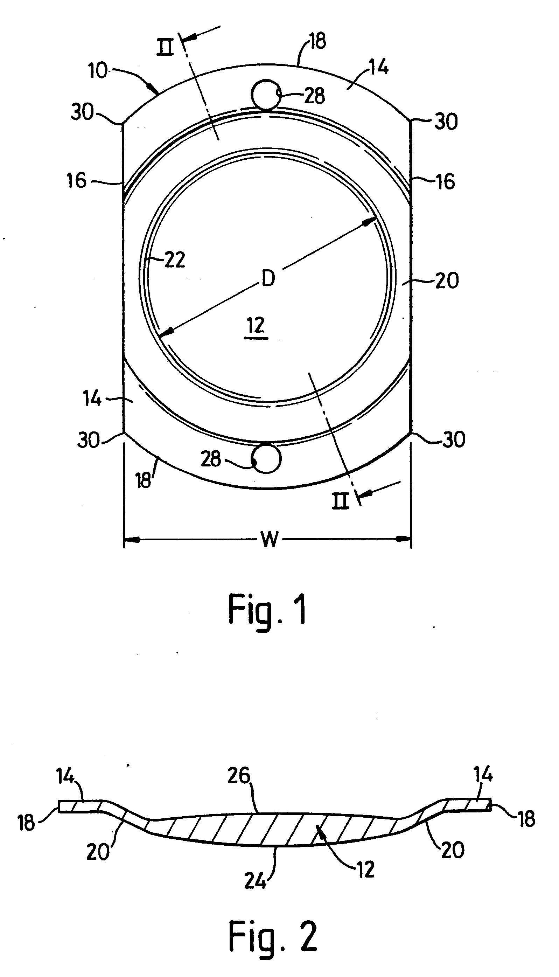

A lens 10 adapted to be implanted intraocularly, embodying the invention, is shown in plan view in Figure 1. The lens 10 comprises an optic 12 which is circular in plan and a haptic which comprises two identical portions 14, positioned at opposite sides of the optic 12. The two portions 14 of the haptic, viewed in plan, are part of an annulus centered on the centre of the optic 12 and defined at opposite ends by parallel chords 16 of the circle defining the outer periphery 18 of the annulus. The haptic further comprises a frusto-conical portion 20 which is annular in plan and extends between inner boundaries of said two portions 14 and the periphery 22 of the optic 12.

As can be seen from Figure 2, the two end portions 14 are provided by substantially planar flanges, and the end portions 14 are vaulted forward of the optic by the frusto-conical portion 20. The posterior surface 24 of the optic 12 has a smaller radius of curvature than the anterior surface 26 which may, indeed, be flat or substantially so.

Holes 28 are provided in the two end portions 14 so that the holes can be engaged by appropriate surgical instruments to facilitate implanting of the lens; if desired a greater number of holes than the two shown in the drawings may be provided.

In the illustrative lens the diameter D of the optic 12 is about 6 mm, the diameter of the periphery 18 is about 10.5 mm and the width W between the chords 16 defining the sides of the two portions 14 of the haptic is about 7 mm. The semi-apical angle of the frusto-conical portion 20 is about 65° and the radial width of the two portions 14 is about 1.25 mm. Although other dimensions may be adopted, it is believed that a lens substantially of the dimensions indicated above will be suitable for use as an implant for most eyes.

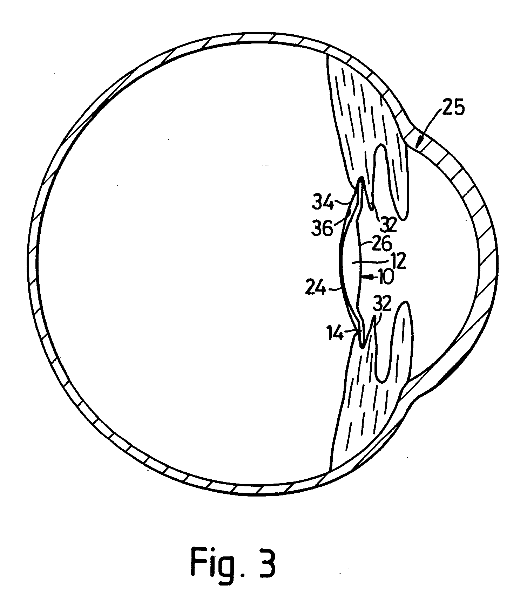

In Figure 3 the illustrative lens is shown in position in an eye. To insert the illustrative lens 10 an incision is made at the upper limbus 25. The lens 10 is manipulated using appropriate surgical instruments with the assistance of the holes 28, through the incision and introduced through the pupil into the remains of the capsular bag through the opening left by removal of the central part of the anterior capsule. The lens 10 is introduced with the two end portions 14 disposed at the upper and lower parts of the eye and retained in place by engagement of the portions 14 between the remains 32 of the anterior capsule and the posterior capsule 34. The posterior surface 24 of the lens is seated firmly against the front surface 36 of the posterior capsule 34.

The illustrative lens 10 is substantially easier to insert than disc lenses and, because the diameter of the periphery 18 can be somewhat greater than is commonly the case with known disc lenses, the tendency for the lens to move in or dislocate forwards from the capsular bag is less than with many disc lenses. Furthermore, because the two portions 14 extend around a considerable part of the optic 12, the possibility of the anterior capsule remains 32 and posterior capsule 34 coming into contact with one another is less than is the case with lenses having flexible haptics or the three limbed type of haptic referred above.

The vaulting of the illustrative lens 10 gives immediately improved contact between the posterior capsule 34 and the optic 12. The substantially uniform pressure exerted by the outer periphery 18 of the two end portions 14 above and below the lens 10 against the remains of the capsular bag also militates against undesirable displacement of the lens, and more uniformly distributes the loading on the remains of the capsular bag stretches the posterior capsule 34 uniformly and prevents creasing of the posterior capsule 34.

Further, the substantially uniform pressure exerted militates against undesirable displacement of the lens, and more uniformly distributes the loading on the remains of the capsular bag than is the case with, for example, the heretofore known type of lens having three limbs.

The planar end portions 14 engage the remains 32 of the anterior capsule to prevent forward displacement of the lens 10.

The illustrative lens is made of a suitable plastics material which is substantially inert and non-irritant for example methylmethacrylate copolymer e.g. that supplied under the designation perspex CQ-UV which also includes an ultraviolet light filtering component.

## Claims

1. A lens (10) adapted to be implanted intraocularly comprising an optic (12) and a haptic (14) characterised in that the haptic comprises two portions (14) positioned at opposite ends of the optic (12) said two portions (14) viewed in plan each being part of an annulus centered on the centre of the optic (12) and being defined at opposite sides by parallel, or substantially parallel chords (16) of the circle defining the outer periphery (18) of the annulus.

2. A lens (10) according to Claim 1 characterised in that the haptic further comprises a portion (20) which is annular in plan and generally frusto-conical, extending between inner boundaries of said two end portions (14) and the periphery (22) of the optic (12).

3. A lens (10) according to either one of Claims 1 and 2 characterised in that the two end portions (14) are substantially planar.

4. A lens (10) according to any one of the preceding claims characterised in that the optic (12) is between 5 and 7 mm in diameter (D).

5. A lens (10) according to any one of the preceding claims characterised in that the diameter of the outer periphery (18) of said end portions (14) is between 9.5 mm and 11.5 mm.

6. A lens (10) according to any one of the preceding claims characterised in that the distance (W) between said chords (16) exceeds the diameter (D) of the optic (12)and is between 6 and 9 mm.

7. A lens (10) according to Claim 2 characterised in that the semi-apical angle of the frusto-conical portion (20) is between 55° and 75°.

8. A lens (10) according to any one of the preceding claims comprising means (20) to facilitate implanting the lens (10).

Fig. 1

Fig. 2

Fig. 3

European Patent Office

EUROPEAN SEARCH REPORT

Application Number

EP 89 31 2353

## DOCUMENTS CONSIDERED TO BE RELEVANT

| Category | Citation of document with indication, where appropriate, of relevant passages | Relevant to claim | CLASSIFICATION OF THE APPLICATION (Int. Cl.5) |
|---|---|---|---|
| X | EP-A-0 136 807 (EZEKIEL NOMINEES)<br>* Page 7, line 22 - page 8, line 5; page 10, lines 10-17; page 11, lines 17-20; page 12, lines 11-13; figures 4a,9,10 *<br>--- | 1-8 | A 61 F   2/16 |
| X | US-A-4 254 509 (TENNANT)<br>* Column 2, lines 33-42; figure 1 *<br>--- | 1 | |
| A | GB-A-2 151 371 (CESKOSLOVENSKA ADAKEMIE VED)<br>* Page 2, lines 24-36; figures 1-3 *<br>----- | 1 | |

TECHNICAL FIELDS SEARCHED (Int. Cl.5)

A 61 F

The present search report has been drawn up for all claims

| Place of search | Date of completion of the search | Examiner |
|---|---|---|
| THE HAGUE | 06-03-1990 | MOERS R.J. |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P0401)